# EUROPEAN PATENT SPECIFICATION

(11) **EP 1 515 699 B1**
(45) Date of publication and mention of the grant of the patent: **04.02.2009**
(21) Application number: 03711920.3
(22) Date of filing: 07.03.2003
(51) Int. Cl.: A61K 9/14, A61K 9/16

(54) **PROCESS FOR LOADING AND THERMODYNAMICALLY ACTIVATING DRUGS ON POLYMERS BY MEANS OF SUPERCRITICAL FLUIDS**
VERFAHREN ZUM LADEN UND THERMODYNAMISCHEN AKTIVIEREN VON ARZNEIMITTELN AUF POLYMEREN MIT SUPERKRITISCHEN FLÜSSIGKEITEN
PROCEDE DE CHARGEMENT ET D'ACTIVATION THERMODYNAMIQUE DE MEDICAMENTS SUR DES POLYMERES AU MOYEN DE FLUIDES SURCRITIQUES

(30) Priority: 07.03.2002 IE 20020184
(43) Date of publication of application: 23.03.2005
(73) Proprietor: EURAND PHARMACEUTICALS LTD., Bray Wicklow (IE)
(72) Inventor: BRESCIANI, Massimo, 34149 Trieste (IT); DOBETTI, Luca, 34149 Trieste (IT); KIRCHMAYER, Stefano, 34143 Trieste (IT)
(74) Representative: Gervasi, Gemma
(86) International application number: PCT/EP2003/002204
(87) International publication number: WO 2003/074028

(56) References cited:
- WO-A-00/69559
- WO-A-01/68054
- WO-A-94/18264
- WO-A-99/25322
- US-A- 4 695 621

## Description

### Field of the invention

The present invention refers to a process by means of the supercritical fluids for loading and thermodynamically.activating drugs on inert polymers.

### Prior art

The technology with supercritical fluids has been developed owing to the particular properties of these fluids for a safer use of them in pharmaceutical field compared to the use of organic solvents.

A supercritical fluid is a material above its temperature and pressure conditions; it exhibits interesting behaviour by combining the properties of conventional liquids and gases. Although their gas-like low viscosities lead to higher rates of flow and diffusion, their liquid-like densities permit higher solvent power. For a detailed description of supercritical fluids, reference can be made to e.g. Kirk-Othmer, Encyclopedia of Chemical Technology, vol.23, p.452-4.53.

The use of supercritical fluids could be, in principle, a valid alternative to the use of solvents in pharmaceutical field. In fact, the supercritical fluids, which are gases in standard environmental conditions, are completely removed from the compounds at the end of the process.

The supercritical fluids are extendedly used to reduce the particle size of drugs and to produce solid particles having a narrow size distribution. This can also be made at mild operating conditions, avoiding the stresses given by other more common techniques (i.e. milling, micronisation). As an example, WO 97/14407 (1. B. Henriksen et al.) deals with the preparation of water-insoluble drugs having an average size from 100 to 300 nm, obtained by dissolving them in a solution and then spraying the solution into supercritical fluid in presence of suitable surface modifiers.

In the last year, the technology with supercritical fluids has been used for loading organic molecules in polymers. M. L. Sand (US Patent 4,598,006) discloses a method for impregnating thermoplastic polymers with additives such as fragrances, pest control agents and pharmaceutical compounds. F. Carli et al. (WO 99/25322) describes the loading of cross-linked polymers with drugs dissolved in supercritical fluids.

Oral delivery of poorly soluble drug has become, in the last years, one of the most challenging problems for advanced pharmaceutical research. This in tum leads to formulations with high drug content which often must be delivered repeatedly to obtain and maintain therapeutic plasma levels.

A way to enhance the solubility of poorly soluble or insoluble drugs is to thermodynamically activate them by forming an amorphous phase and/or nanocrystalline structures from the original crystalline state. This results in drug solubilisation kinetic, having dissolution rate and supersaturation concentrations, that is much higher than that obtainable with differently formulated drug in crystalline state. As a consequence, a strong increase of the drug effects "in-vivo" is allowed by enhancing the bioavailability, reducing the onset of action (tₘₐₓ) and decreasing the variability between subjects.

The process described in WO 99/25322 has then been applied to check the suitability of supercritical fluids for the thermodynamic activation of drugs in cross-linked polymers. Positive results in terms of drug activation have been obtained. Now, we have surprisingly found that a pre-treatment of the cross-linked polymer with pure supercritical fluid allows a higher degree and a more rapid kinetic of drug loading into cross-linked polymers (shorter process time) when compared to a standard process without pre-treatment. Moreover, a hither thermodynamic activation of the drugs is also obtained by means of a pre-treatment step.

### Summary of the invention

The present invention refer to a process of loading drugs in a thermodynamic activated form into polymers by means of supercritical fluids. The process includes a pre-treatment step of the cross-linked polymer with pure supercritical fluid to allow a higher degree and a more rapid kinetic of drug loading into cross-linked polymers and also a higher thermodynamic activation of the drugs.

### Detailed description of the invention

Object of the present invention is a process to load drugs into cross-linked polymers by means of supercritical fluids. The process includes a pre-treatment step of a cross-linked polymer with a supercritical fluid; this process allows to obtain a higher degree and a more rapid kinetic of drug loading into cross-linked polymers (shorter process time) and also a higher thermodynamic activation of the drugs.

The supercritical fluid used in the pre-treatment step is free from any drugs (hereinafter referred as "pure supercritical fluid"); the pure supercritical fluid as such can be produced by means known in the art, i.e. by compressing the fluid and passing it through a heat exchanger in order to bring it beyond those temperature and pressure values at which it forms a supercritical fluid. Non limiting examples of substances from which supercritical fluids can be obtained are carbon dioxide, hydrocarbon (ethylene, propylene), chlorofluorocarbon, nitrous oxide; supercritical fluids can be used alone or as a mixture of more of them.

In the pre-treatment step, the pure supercritical fluid is pumped into a reactor containing the pure cross-linked polymer (i.e. the polymer not containing any drugs) and is maintained in supercritical conditions of temperature and pressure; the contact time between pure supercritical fluid and pure polymer is preferably between 1 minutes and 6 hours, most preferably between 5 minutes and 4 hours. The thus pre-treated polymer can be discharged from the reactor (after removing the supercritical fluid) and preserved for later loading with a drug, or can be immediately loaded with the drug. In both cases, the drug-loading step can be effected by contacting the polymer with an aliquot of supercritical fluid containing the drug dissolved therein (this solution can be formed e.g. by passing a supercritical -fluid through an extractor containing the drug to be solubilised) and pumping this solution into the reactor containing the cross-linked polymer, maintained in suitable supercritical conditions of temperature and pressure. The contact time of the supercritical fluid containing the solubilised drug with the polymer is preferably between 2 minutes and 48 hours, most preferably between 10 minutes and 12 hours.

The contact between polymer and fluid, for both pre-treatment and drug-loading step, can be carried out in static or dynamic conditions or in a combination of them. In the static case, a predetermined volume of supercritical fluid, with (drug-loading step) or without (pre-treatment step) the solubilised drug, is introduced in a container and allowed to equilibrate in contact with the polymer. In the dynamic process, the stream of supercritical fluid, generated by the pump at the outlet of the extractor, is passed through a column containing the polymer. The combined process, static plus dynamic, can be obtained, for example, by passing dynamically a volume of supercritical fluid without the solubilised drug, through a column, by stopping the stream, leaving the supercritical fluid in contact with polymer in static conditions, and then passing again the supercritical fluid with the solubilised drug through the column, and leaving the supercritical fluid in contact with polymer in static conditions.

During both pre-treatment and drug-loading steps, pressure and temperature are maintained controlled, preferably constant, so as to maintain the fluid inside the reactor in supercritical conditions: this can be done by suitably using heat exchangers, constant monitoring of the pressure, and releasing controlled amounts of supercritical fluid when fresh fluid is added into the reactor.

At the outlet of the reactor, the fluid stream is passed through an absorber suitable to remove from the stream any traces of the residual drug. The fluid stream is then brought back to the ambient conditions and drained or, if necessary, cooled, sent to a reflux receiver and recycled.

The addition of the drug-loaded supercritical fluid results with the polymer structure being filled with the a supercritical solution of the drug. After the drug-loading phase, the supercritical fluid is removed from the reactor, causing the dissolved drug to precipitate in microparticle form inside the cross-linked polymeric network; the removal of the supercritical fluid can be conveniently effected by decreasing the pressure (and/or increasing the temperature) inside the reactor, thereby allowing the fluid to evaporate in gaseous form; when the concentration of drug increases over the solubility value in the fluid, the drug starts precipitating into the polymeric network; the total removal of the fluid leaves a solid powder in the reactor consisting of the drug-loaded polymer.

Cross-linked polymers useful for the present invention are any polymers (hydrophilic, hydrophobic or amphiphilic), whose polymeric chains are cross-linked by interchain bonds: these bonds can be naturally present in the polymer as such, or can be added by performing ad-hoc cross-linking reactions: as known in the art, cross-linking can be obtained by polymerisation processes that produces physically crosslinked polymers, or by a chemical reaction of linear polymers with crosslinking agents. Not exhaustive examples of cross-linked polymer useful for the present invention are: cross-linked polyvinylpyrrolidone, cross-linked cellulose derivatives such as sodium croscarmellose, starch and its derivatives such as sodium starch glycolate, cyclodextrins and their derivatives, cross-linked polystyrene and cross-linked acrylic polymers. Cross-linked polymers can be used alone or as a mixture of more of them.

The cross-linked polymer loaded with this process contains preferably from 0.5% to 70%, more preferably from 3% to 50%, by weight of the active drug to the final total mass (cross-linked polymer + loaded drug). Any drugs which can be solubilised into the supercritical fluid can be used for the purpose of the present invention. Among the drugs which can be loaded and activated according to the process of the invention, not exhaustive examples are Cox-2 inhibitors, anti-inflammatory drugs such as nimesulide, piroxicam, naproxene, ketoprofen, ibuprofen and diacerhein, anti-fungal drugs such as griseofulvin, itraconazole, fluconazole, miconazole and ketoconazole, bronchodilators/anti-asthmatic drugs such as zafirlukast, salbutamol, beclomethasone, flunisolide, clenbuterol, salmeterol and budesonide, steroids such as estradiol, estriol, progesterone, megestrol acetate and medroxyprogesterone acetate, anti-hypertensive/anti-thrombotic/vasodilator drugs such as nifedipine, nicergoline, nicardipine, lisinopril, enalapril, nicorandil, celiprolol and verapamil, benzodiazepines such as temazepam, diazepam, lorazepam, fluidiazepam, medazepam and oxazolam, anti-migraine drugs such as zolmitriptan and sumatriptan, anti-hyperlipoproteinemic drugs such as fenofibrate, lovastatin, atorvastatin, fluvastatin and simvastatin, anti-viral/anti-bacterial drugs such as tosufloxacin, ciprofloxacin, ritonavir, saquinavir, nelfinavir, acyclovir and indinavir, immunodepressant drugs such as tacrolimus, rapamycin and didanisine, anti-histaminic drugs such as loratidine, anti-thumoral drugs such as etoposide, bicalutamide, tamoxifen, doclitaxel and paclitaxel, anty-psycotic drugs such as risperidone, anti-osteoporotic drugs such as raloxifene, anti-convulsant such as carbamazepine, analgesic/narcotic drugs such as oxycodone, hydrocodone, morphine and butorpanol, muscle relaxant such as tinazadine, anti-convulsant drug such as phenytoin, anti-ulcerative drugs such as famotidine.

The present inventors have found that when a cross-linked polymer is treated with a supercritical fluid according to the pre-treatment described above, it can be loaded with much higher amounts of drug than in the case of the untreated polymer: it is believed that the pre-treatment with the supercritical fluid (not containing any drugs) operates a chemical-physical modification in the polymer network, making it more prone to capture the drug particles in a subsequent drug-loading process: this fact is confirmed in the experimental part, where it is shown that a drug loading process by means of supercritical fluids results in a significantly higher percentage of drug incorporation if, in place of a common cross-linked polymer, the cross-linked polymer pre-treated in accordance with the pre-treatment of the invention is used.

In accordance with the above findings, the present invention also embraces a method to increase the drug-loading capacity of a cross-linked polymer, characterised by treating said cross-linked polymer with a supercritical fluid not containing any drugs. A further consequent object of the invention is a modified cross-linked polymer, having an enhanced capacity to incorporate drugs, obtained by treating a cross-linked polymer with a supercritical fluid not containing any drugs, in the modalities hereabove described.

A further surprising finding is that the drug incorporated in the polymer according to the present process shows an increased amount in its highly bioavailable amorphous and nanocrystalline fractions. The increase in the amorphous/nanocrystattine fraction obtains an increased biovailability of the drug, due to the much quicker solubility of these forms with respect to the crystalline one.

In accordance with the above findings, the invention also comprises a method to increase the amorphous/nanocrystalline fraction of a drug (or to reduce its crystalline fraction and thereby increasing its activation degree), characterised by: (a) pre-treating a cross-linked polymer with a supercritical fluid; (b) contacting said pre-treated polymer with a supercritical fluid containing the drug dissolved therein; (c) removing the supercritical fluid, which results in the drug being precipitated inside the cross-linked polymer in an increased amorphous/nanocrystalline fraction.

The process of the invention allows for the first time to incorporate large amounts of drugs into cross-linked polymers while, at the same time increasing substantially the bioavailability of the incorporated drug. Consequently, new highly potent pharmaceutical compositions can be obtained, associating a high drug content with an enhanced bioavailability of the same. These pharmaceutical compositions are also within the scope of the present invention.

The invention is further illustrated with reference to the following non-imitative examples.

### EXPERIMENTALS

The presence of amorphous, nanocrystalline or crystal phase can be detected by means of Differential Scanning Calorimetry (DSC). Compared to the sharp melting peak of the drug crystal, the nanocrystals present a broader peak with a markedly - lower maximum of temperature (I. Colombo-et.al. 4th lnt. Conf. Pharm. Technol., 1986; F. Carli et al. Acta Pharm. Jugosl. 38, 361, 1988). The amorphous phase does not show any thermal event.

In the examples, the activation level is expressed as the fraction of crystalline form. It is determined by comparing the enthalpy relative to the melting of the crystals in the polymer (AH_{melting}) to that of pure drug (ΔH₀). The AH_{melting}/AH₀ ratio, normalized in accordance with the drug assayed in the polymer, is then considered equal to the fraction of crystalline form. The higher the amount of crystals (higher crystallinity), the lower the thermodynamic activation level of the drug.

### Example 1

### Reference (1R)

5 g of cross-linked polyvinylpyrrolidone, placed in a cylindrical reactor of 50 cm length and 0.6 cm diameter, are contacted for 8 hours with a 450 mL/min stream of supercritical in carbon dioxide (CO₂) saturated with nimesulide. Temperature and pressure are 40°C and 130 bar, respectively.

### Invention (1I)

5 g of cross-linked polyvinylpyrrolidone, placed in a cylindrical reactor of 50 cm length and 0.6 cm diameter, are pre-treated for 30 minutes with a 450 mL/min stream of supercritical in carbon dioxide (CO₂). The cross-linked polymer is then contacted for 6 hours with a 450 mL/min stream of supercritical in carbon dioxide (CO₂) saturated with nimesulide. Temperature and pressure of both pre-treatment and loading steps are 40°C and 130 bar, respectively.

The results, reported in Table 1, show a higher degree of drug loading, a shorter process time (even considering the pre-treatment step) and a higher activation level (lower crystallinity) in the example 1I of the invention compared to the reference 1 R of prior art.

**Table 1.**

| | Drug content **(%)** | **T_{melting} (°C)** | Δ**H_{meltlng} (J/g)** | **Crystallinity (%)** |
|---|---|---|---|---|
| Nimesulide | - | 148.4 | 109.2 | 100 |
| 1R | 8.2 | 147.9 | 1.5 | 17 |
| 1I | 9.1 | no peak | - | 0 |

### Example 2

### Reference (2R)

5 g of cross-linked polyvinylpyrrolidone, placed in a cylindrical reactor of 50 cm length and 0.6 cm diameter, are contacted for 8 hours with a 450 mL/min stream of supercritical in carbon dioxide (CO₂) saturated with ibuprofen. Temperature and pressure are 40°C and 130 bar, respectively.

### Invention (2I)

5 g of cross-linked polyvinylpyrrolidone, placed in a cylindrical reactor of 50 cm length and 0.6 cm diameter, are pre-treated for 30 minutes with a 450 mL/min stream of supercritical in carbon dioxide (CO₂). The cross linked polymer is then contacted for 6 hours with a 450 mL/min stream of supercritical in carbon dioxide (CO₂) saturated with ibuprofen. Temperature and pressure of both pre-treatment and loading steps are 40°C and 130 bar, respectively:

The results, reported in Table 2, show a higher degree of drug loading, a shorter process time (even considering the pre-treatment step) and a higher activation level (lower crystallinity) in the example 2l of the invention compared to the reference 2R of prior art.

**Table 2.**

| | Drug content **(%)** | **T_{melting} (°C)** | Δ**H_{melting} (J/g)** | **Crystallinity (%)** |
|---|---|---|---|---|
| Ibuprofen | - | 75.1 | 22.9 | 100 |
| 1 R | 10.5 | 74.4 | 4.4 | 34 |
| 1 I | 16.0 | no peak | - | |

### Example 3

### Reference (3R)

5 g of cross-linked polyvinylpyrrolidone, placed in a cylindrical reactor of 50 cm length and 0.6 cm diameter, are contacted for 8 hours with a 450 mL/min stream of supercritical in ethylene saturated with ibuprofen. Temperature and pressure are 30°C and 120 bar, respectively.

### Invention (3I)

5 g of cross-linked polyvinylpyrrolidone, placed in a cylindrical reactor of 50 cm length and 0.6 cm diameter, are pre-treated for 30 minutes with a 450 mL/min stream of supercritical in ethylene. The cross-linked polymer is then contacted for 6 hours with a 450 mL/min stream of supercritical in ethylene saturated with ibuprofen. Temperature and pressure of both pre-treatment and loading steps are 40°C and 130 bar, respectively.

The results, reported in Table 3, show a higher degree of drug loading, a shorter process time (even considering the pre-treatment step) and a higher activation level (lower crystallinity) in the example 3I of the invention compared to the reference 3R of prior art.

**Table 3.**

| | Drug content **(%)** | **T_{melting} (°C)** | Δ**H_{melting} (J/g)** | **Crystallinity (%)** |
|---|---|---|---|---|
| Ibuprofen | - | 75.1 | 122.9 | 100 |
| 1R | 6.4 | 74.0 | 7.2 | 92 |
| 1I | 7.9 | 74.2 | 2.3 | 24 |

## Claims

1. A process to load a drug into a cross-linked polymer, comprising the following steps:
a. pre-treating said cross-linked polymer with a supercritical fluid;
b. contacting said pre-treated cross-linked polymer with a supercritical fluid containing the drug dissolved therein;
c. removing the supercritical fluid, thereby causing the drug to precipitate inside the cross-linked polymers.

2. Process according to claim 1, wherein in step a. , the cross-linked polymer is maintained in contact with the supercritical fluid for a time comprised between 1 minute and 6 hours.

3. Process according to claims 1-2, wherein in step a. , the cross-linked polymer is maintained in contact with the supercritical fluid for a time comprised between 5 minutes and 4 hours.

4. Process according to claims 1-3, wherein in step b. , the pre-treated cross-linked polymer is maintained in contact with the supercritical fluid for a time comprised between 2 minutes and 48 hours.

5. Process according to claims 1-4, wherein in step b. , the pre-treated cross-linked polymer is maintained in contact with the supercritical fluid for a time comprised between minutes and 12 hours.

6. Process according to claims 1-5, wherein the contact of the cross-linked polymer with the supercritical fluid is effected in static and/or dynamic conditions.

7. Process according to claims 1-6, wherein said supercritical fluid is chosen among carbon dioxide, ethylene, propylene, chlorofluorocarbon, nitrous oxide, and mixtures thereof.

8. Process according to claims 1-7, wherein said cross-linked polymer is chosen among cross-linked polyvinylpyrrolidone, cross-linked cellulose derivatives, starch and its derivatives, cyclodextrins and their derivatives, cross-linked polystyrene, cross-linked acrylic polymers, and mixtures thereof.

9. Process according to claims 1-8, further **characterised in that** the thus loaded drug is present in the cross-linked polymer in high amorphous and nanocrystalline fraction.

10. A method to increase the drug-loading capacity of a cross-linked polymer, consisting in treating said cross-linked polymer with a supercritical fluid not containing any drugs.

11. Method according to claim 10, wherein the cross-linked polymer is maintained in contact with the supercritical fluid for a time comprised between 1 minute and 6 hours.

12. Method according to claims 10-11, wherein the cross-linked polymer is maintained in contact with the supercritical fluid for a time comprised between 5 minutes and 4 hours.

13. Method according to claims 10-12, wherein the contact of the polymer with the supercritical fluid is effected in static and/or dynamic conditions.

14. Method according to claims 10-13, wherein the supercritical fluid is chosen among carbon dioxide, ethylene, propylene, chlorofluorocarbon, nitrous oxide, and mixtures thereof.

15. Method according to claims 10-14, wherein the cross-linked polymer is chosen among cross-linked polyvinylpyrrolidone, cross-linked cellulose derivatives, starch and its derivatives, cyclodextrins and their derivatives, cross-linked polystyrene, cross-linked acrylic polymers, and mixtures thereof.

16. Modified cross-linked polymer, having enhanced drug-loading properties, obtainable from a polymer selected from the group consisting of cross-linked polyvinylpirrolidone, cross-linked cellulose derivatives, starch and its deivatives, cyclodextrins and their derivatives, cross-linked polystyrene and mixtures thereof by treating the sole cross-linked polymer with a supercritical fluid not containing any drug.

17. Modified cross-linked polymer according to claim 16, obtainable by treating the sole cross-linked polymer with the supercritical fluid for a time comprised between 1 minute and 6 hours.

18. Modified cross-ilnked polymer according to claims 16 or 17, obtainable by treating the sole cross-linked polymer with the supercritical fluid for a time comprised between 5 minutes and 4 hours.

19. Modified cross-linked polymers according to claims 16-18, wherein the supercritical fluid is chosen among carbon dioxide, ethylene, propylene, chlorofluorocarbon, nitrous oxide, and mixtures thereof.

20. Modified cross-linked polymer according go claims 16-19, loaded with a drug.

21. Pharmaceutical composition containing a modified cross-linked polymer according to claim 20.

## Patentansprüche

1. Verfahren zum Beladen eines vernetzten Polymers mit einem Wirkstoff, das die folgenden Schritte umfaßt:
a. Vorbehandeln des vernetzten Polymers mit einer überkritischen Flüssigkeit;
b. Inkontaktbringen des vorbehandelten vernetzten Polymers mit einer überkritischen Flüssigkeit, die den darin gelösten Wirkstoff enthält;
c. Entfernen der überkritischen Flüssigkeit, wodurch der Wirkstoff im Inneren des vernetzten Polymers zum Präzipitieren gebracht wird.

2. Verfahren gemäß Anspruch 1, worin in Schritt a. das vernetzte Polymer mit der überkritischen Flüssigkeit über eine Zeit zwischen 1 Minute und 6 Stunden in Kontakt gehalten wird.

3. Verfahren gemäß Ansprüchen 1 bis 2, worin in Schritt a. das vernetzte Polymer mit der überkritischen Flüssigkeit über eine Zeit zwischen 5 Minuten und 4 Stunden in Kontakt gehalten wird.

4. Verfahren gemäß Ansprüchen 1 bis 3, worin in Schritt b. das vorbehandelte vernetzte Polymer mit der überkritischen Flüssigkeit über eine Zeit zwischen 2 Minuten und 48 Stunden in Kontakt gehalten wird.

5. Verfahren gemäß Ansprüchen 1 bis 4, worin in Schritt b. das vorbehandelte vernetzte Polymer mit der überkritischen Flüssigkeit über eine Zeit zwischen 10 Minuten und 12 Stunden in Kontakt gehalten wird.

6. Verfahren gemäß Ansprüchen 1 bis 5, worin der Kontakt des vernetzten Polymers mit der überkritischen Flüssigkeit unter statischen und/oder dynamischen Bedingungen bewirkt wird.

7. Verfahren gemäß Ansprüchen 1 bis 6, worin die überkritische Flüssigkeit aus Kohlendioxid, Ethylen, Propylen, Chlorfluorkohlenstoff, Stickstoffoxid und Mischungen daraus ausgewählt ist.

8. Verfahren gemäß Ansprüchen 1 bis 7, worin das vernetzte Polymer aus vernetztem Polyvinylpyrrolidon, vernetzten Cellulosederivaten, Stärke und deren Derivaten, Cyclodextrinen und deren Derivaten, vernetztem Polystyrol, vernetzten Acrylpolymeren und Mischungen daraus ausgewählt ist.

9. Verfahren gemäß Ansprüchen 1 bis 8, weiterhin **dadurch gekennzeichnet, daß** der so geladene Wirkstoff im vernetzten Polymer in hohem amorphem und nanokristallinem Anteil vorhanden ist.

10. Verfahren zum Erhöhen der Wirkstoffbeladungskapazität eines vernetzten Polymers, das im Behandeln des vernetzten Polymers mit einer überkritischen Flüssigkeit, die keine Wirkstoffe enthält, besteht.

11. Verfahren gemäß Anspruch 10, worin das vernetzte Polymer mit der überkritischen Flüssigkeit über eine Zeit zwischen 1 Minute und 6 Stunden in Kontakt gehalten wird.

12. Verfahren gemäß Ansprüchen 10 bis 11, worin das vernetzte Polymer mit der überkritischen Flüssigkeit über eine Zeit zwischen 5 Minuten und 4 Stunden in Kontakt gehalten wird.

13. Verfahren gemäß Ansprüchen 10 bis 12, worin der Kontakt des Polymers mit der überkritischen Flüssigkeit unter statischen und/oder dynamischen Bedingungen bewirkt wird.

14. Verfahren gemäß Ansprüchen 10 bis 13, worin die überkritische Flüssigkeit aus Kohlendioxid, Ethylen, Propylen, Chlorfluorkohlenstoff, Stickstoffoxid und Mischungen daraus ausgewählt ist.

15. Verfahren gemäß Ansprüchen 10 bis 14, worin das vernetzte Polymer aus vernetztem Polyvinylpyrrolidon, vernetzten Cellulosederivaten, Stärke und deren Derivaten, Cyclodextrinen und deren Derivaten, vernetztem Polystyrol, vernetzten Acrylpolymeren und Mischungen daraus ausgewählt ist.

16. Modifiziertes vernetztes Polymer mit verbesserten Wirkstoffbeladungseigenschaften, das aus einem Polymer, das aus der Gruppe ausgewählt ist, die aus vernetztem Polyvinylpyrrolidon, vernetzten Cellulosederivaten, Stärke und deren Derivaten, Cyclodextrinen und deren Derivaten, vernetztem Polystyrol und Mischungen daraus besteht, durch Behandeln des bloßen vernetzten Polymers mit einer überkritischen Flüssigkeit, die keinen Wirkstoff enthält, erhältlich ist.

17. Modifiziertes vernetztes Polymer gemäß Anspruch 16, das durch Behandeln des bloßen vernetzten Polymers mit der überkritischen Flüssigkeit über eine Zeit zwischen 1 Minute und 6 Stunden erhältlich ist.

18. Modifiziertes vernetztes Polymer gemäß Ansprüchen 16 oder 17, das durch Behandeln des bloßen vernetzten Polymers mit der überkritischen Flüssigkeit über eine Zeit zwischen 5 Minuten und 4 Stunden erhältlich ist.

19. Modifiziertes vernetztes Polymer gemäß Ansprüchen 16 bis 18, worin die überkritische Flüssigkeit aus Kohlendioxid, Ethylen, Propylen, Chlorfluorkohlenstoff, Stickstoffoxid und Mischungen daraus ausgewählt ist.

20. Modifiziertes vernetztes Polymer gemäß Ansprüchen 16 bis 19, das mit einem Wirkstoff beladen ist.

21. Pharmazeutische Zusammensetzung, die ein modifiziertes vernetztes Polymer gemäß Anspruch 20 enthält.

## Revendications

1. Procédé de chargement d'un médicament dans un polymère réticulé, comprenant les étapes suivantes :
a. prétraitement dudit polymère réticulé avec un fluide supercritique ;
b. mise en contact dudit polymère prétraité réticulé avec un fluide supercritique contenant le médicament dissous dans celui-ci ;
c. retrait du fluide supercritique, entraînant ainsi la précipitation du médicament dans le polymère réticulé.

2. Procédé selon la revendication 1, dans lequel à l'étape a, le polymère réticulé est maintenu en contact avec le fluide supercritique pendant une durée comprise entre 1 minute et 6 heures.

3. Procédé selon les revendications 1 à 2, dans lequel à l'étape a, le polymère réticulé est maintenu en contact avec le fluide supercritique pendant une durée comprise entre 5 minutes et 4 heures.

4. Procédé selon les revendications 1 à 3, dans lequel à l'étape b, le polymère réticulé prétraité est maintenu en contact avec le fluide supercritique pendant une durée comprise entre 2 minutes et 48 heures.

5. Procédé selon les revendications 1 à 4, dans lequel à l'étape b, le polymère réticulé prétraité est maintenu en contact avec le fluide supercritique pendant une durée comprise entre 10 minutes et 12 heures.

6. Procédé selon les revendications 1 à 5, dans lequel le contact du polymère réticulé avec le fluide supercritique s'effectue dans des conditions statiques et/ou dynamiques.

7. Procédé selon les revendications 1 à 6, dans lequel ledit fluide supercritique est choisi parmi le dioxyde de carbone, l'éthylène, le propylène, le chlorofluorocarbone, l'oxyde nitreux et leurs mélanges.

8. Procédé selon les revendications 1 à 7, dans lequel ledit polymère réticulé est choisi parmi la polyvinylpyrrolidone réticulée, les dérivés de cellulose réticulés, l'amidon et ses dérivés, les cyclodextrines et leurs dérivés, le polystyrène réticulé, les polymères acryliques réticulés et leurs mélanges.

9. Procédé selon les revendications 1 à 8, **caractérisé en outre en ce que** le médicament ainsi chargé est présent dans le polymère réticulé dans une fraction hautement amorphe et nanocristalline.

10. Procédé destiné à augmenter la capacité de chargement d'un médicament d'un polymère réticulé consistant à traiter ledit polymère réticulé avec un fluide supercritique ne contenant pas de médicament.

11. Procédé selon la revendication 10, dans lequel le polymère réticulé est maintenu en contact avec le fluide supercritique pendant une durée comprise entre 1 minute et 6 heures.

12. Procédé selon les revendications 10-11, dans lequel le polymère réticulé est maintenu en contact avec le fluide supercritique pendant une durée comprise entre 5 minutes et 4 heures.

13. Procédé selon les revendications 10 à 12, dans lequel le contact du polymère avec le fluide supercritique s'effectue dans des conditions statiques et/ou dynamiques.

14. Procédé selon les revendications 10 à 13, dans lequel ledit fluide supercritique est choisi parmi le dioxyde de carbone, l'éthylène, le propylène, le chlorofluorocarbone, l'oxyde nitreux et leurs mélanges.

15. Procédé selon les revendications 10 à 14, dans lequel le polymère réticulé est choisi parmi la polyvinylpyrrolidone réticulée, les dérivés de cellulose réticulés, l'amidon et ses dérivés, les cyclodextrines et leurs dérivés, le polystyrène réticulé, les polymères acryliques réticulés et leurs mélanges.

16. Polymère réticulé modifié, ayant des propriétés améliorées de chargement de médicament, pouvant être obtenu à partir d'un polymère choisi dans le groupe comprenant la polyvinylpyrrolidone réticulée, les dérivés de cellulose réticulés, l'amidon et ses dérivés, les cyclodextrines et leurs dérivés, le polystyrène réticulé et leurs mélanges par traitement du polymère réticulé seul avec un fluide supercritique ne contenant pas de médicament.

17. Polymère réticulé modifié selon la revendication 16, pouvant être obtenu par traitement du polymère réticulé seul avec le fluide supercritique pendant une durée comprise entre 1 minute et 6 heures.

18. Polymère réticulé modifié selon les revendications 16 ou 17, pouvant être obtenu par traitement du polymère réticulé seul avec le fluide supercritique pendant une durée comprise entre 5 minutes et 4 heures.

19. Polymère réticulé modifié selon les revendications 16 à 18, dans lequel le fluide supercritique est choisi parmi le dioxyde de carbone, l'éthylène, le propylène, le chlorofluorocarbone, l'oxyde nitreux et leurs mélanges.

20. Polymère réticulé modifié selon les revendications 16 à 19, chargé avec un médicament.

21. Composition pharmaceutique contenant un polymère réticulé modifié selon la revendication 20.
